(19) Europäisches Patentamt
European Patent Office
Office européen des brevets

(11) EP 4 349 843 A1

(12) EUROPEAN PATENT APPLICATION
published in accordance with Art. 153(4) EPC

(43) Date of publication:
**10.04.2024 Bulletin 2024/15**

(21) Application number: **22892020.3**

(22) Date of filing: **09.11.2022**

(51) International Patent Classification (IPC):
***C07H 15/18*** *(2006.01)* ***C12P 19/44*** *(2006.01)*
***A61K 8/60*** *(2006.01)* ***C12R 1/06*** *(2006.01)*
***C12N 9/10*** *(2006.01)*

(52) Cooperative Patent Classification (CPC):
**A61K 8/60; A61P 39/06; A61Q 19/02; A61Q 19/08; C07H 15/18; C12N 9/10; C12P 19/18; C12P 19/44;**
C12R 2001/06

(86) International application number:
**PCT/CN2022/130955**

(87) International publication number:
**WO 2023/083226 (19.05.2023 Gazette 2023/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.11.2021 CN 202111328730**
**02.11.2022 CN 202211359878**

(71) Applicant: **Shandong Henglu Biotech. Co., Ltd.**
**Jinan, Shandong 250004 (CN)**

(72) Inventors:
- **HIROSE, Yoshihiko**
  **Ogaki, Gifu 503-0097 (JP)**
- **UEDA, Makoto**
  **Oyama City, Tochigi 323-0806 (JP)**
- **FANG, Xu**
  **Jinan, Shandong 250004 (CN)**
- **YAN, Bingqiang**
  **Jinan, Shandong 250004 (CN)**
- **YIN, Wencheng**
  **Jinan, Shandong 250004 (CN)**
- **LIU, Daming**
  **Jinan, Shandong 250004 (CN)**
- **ZHANG, Shuang**
  **Jinan, Shandong 250004 (CN)**

(74) Representative: **Herrmann, Uwe**
**Lorenz Seidler Gossel**
**Rechtsanwälte Patentanwälte**
**Partnerschaft mbB**
**Widenmayerstraße 23**
**80538 München (DE)**

# (54) ALPHA-SALIDROSIDE, AND PREPARATION METHOD THEREFOR AND APPLICATION THEREOF

(57) The present invention relates to α-salidroside, and a preparation method therefor and an application thereof. The present invention provides novel salidroside, i.e., α-salidroside. Compared with known β-salidroside, in terms of scavenging DPPH free radicals and hydroxyl free radicals, α-salidroside has activity better than that of β-salidroside and can be added to cosmetics or anti-fatigue healthcare products as a new functional ingredient. In the preparation method for α-salidroside of the present invention, by using cheap starch, etc. as a glycosyl donor and tyrosol as a substrate, under the action of a glycosyltransferase, α-salidroside is synthesized. The glycosyltransferase has strict specificity and chiral catalytic capability, and can specifically generate α-salidroside.



Processed by Luminess, 75001 PARIS (FR)

## Description

The present invention claims the priority of Chinese patent applications filed with the China National Intellectual Property Administration on November 10, 2021, with an application number 202111328730.0, titled "α-Salidroside, method for preparing same, and application thereof', and on November 2, 2022, with an application number 202211359878.5, titled "α-Salidroside, method for preparing same, and application thereof. The entire contents of both applications are incorporated herein by reference and constitute a part of the present invention.

## TECHNICAL FIELD

The present invention pertains to α-salidroside, method for preparing same, and application thereof, more specifically, to a method for synthesizing α-salidroside employing glycosyltransferase, belonging to the field of biotechnology.

## BACKGROUND

Rhodiola is a precious herb in traditional Chinese medicine, listed in the 2020 edition of the 'Pharmacopoeia of the People's Republic of China' as the dried roots and rhizomes of *Rhodiola crenulata* (scientific name: *Rhodiola crenulata* (Hook.f.et Thoms.) H.Ohba), belonging to the family Crassulaceae. It is found in regions like Tibet in China, growing in areas with altitudes ranging from 4,050 meters to about 5,400 meters. Medicinal products derived from Rhodiola, such as Rhodiola tablets and Xin Nao Xin capsules, etc., possess ameliorating effect of blood circulation, removing vascular blockage, dredging collateral, and alleviating pain. Health supplements, including Rhodiola capsules, etc., boost immunity and relieve physical fatigue. Salidroside is a main active ingredient in the traditional Chinese medicinal herb Rhodiola, which has been reported to have various biological activities, including protecting cardiovascular and cerebrovascular systems, anti-fatigue, anti-depression, anti-aging, anti-hypoxia, anti-radiation, anti-tumor, immune regulation, skin whitening and freckle removal, etc. The conformation of salidroside (plant derived) is β-glucopyranoside. The chemical formula is as follows:

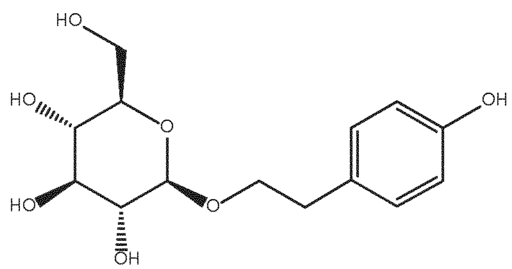

**Chemical structure of β-salidroside.**

The H-NMR spectrum of β-salidroside: 1H-NMR (CDsOD), δ ppm: 7.06 (2H, d, J=8.2 Hz, H-2 and, H-6), 6.70 (2H, d, J=8.2 Hz, H-3 and, H-5), 2.82 (2H, t, J=7.2 Hz, H-α), 3.70 and 4.04 (d, representing one hydrogen on the β-$CH_2$, respectively), 4.28 (1H, d, glycosidic proton), 3.68 and 3.84 (d, representing two hydrogens on the 6'-$CH_2$, respectively), 3.1-3.4 (4H, The H-NMR spectrum of β-salidroside m, representing H-2', H-3', H-4', and H-5', among which δ3.24 ppm is for H-2', δ3.27 ppm is for H-4', 63.25-3.44 ppm are for H-3' and H-5').

The C-NMR spectrum of β-salidroside: 13C-NMR ($CD_3OD$), δppm: 36.37 ( α-C), 72.09 (β-C), 62.75 (C-6'), 78.10(C-5'), 71.64(C-4'), 75.11(C-2'), 77.96(C-3'), 104.37(C-1'), 78.10(C-5'), 71.64(C-4'), 75.11(C-2'), 77.96(C-3'), 104.37(C-1'), 130.93 (C-1), 156.81 (C-4), 116.11 (C-3 and C-5), 130.93 (C-2 and C-6).

Due to the limited resources of Rhodiola, some researchers have conducted research on the preparation of salidroside by plant extraction, enzymatic synthesis, biosynthesis, chemical synthesis, etc. Chinese patent document CN104774815A (application number 201510160496.3) disclosed a glycosyltransferase catalyzing the synthesis of gastrodin or salidroside. The glycosyltransferase in this patent was derived from the glycosyltransferase gene ugt73b6 of *Rhodiola sachalinensis,* which, after undergoing error-PCR random mutation, resulted in the mutant glucosyltransferase gene ugt73b6$^{MK}$ catalyzing the synthesis of gastrodin or salidroside. Specifically, the methionine at position 264 was mutated to lysine. Chinese patent CN108220264A (application number 201611200315.6) disclosed a glycosyltransferase and its application in the biosynthesis of β-salidroside. The glycosyltransferase in this patent was derived from the UDP-glycosyltransferase of *Arabidopsis thaliana* (Genbank GI: 66774038). Chinese patent CN106543243 (application number 201610979792.0) disclosed a salidroside derivative and its preparation method. The glycosyltransferase used in this

patent was derived from the cyclodextrin glucosyltransferase of *Bacillus subtilis* ATCC 6699. Chinese patent CN106543244A (application number 201610979793.5) disclosed the use of galactosidase to prepare β-galactosyl salidroside and its derivatives. The galactosidase used in this patent was the β-galactosidase from *Enterobacter cloacae.* Chinese patent CN109321615A (application number 201811363071.2) disclosed the application of *Bacillus amyloliquefaciens* with high glycosyltransferase activity in the biosynthesis of salidroside in non-aqueous phase. In this patent, the wet cells of *Bacillus amyloliquefaciens* FJ18 proceeded the glycosylation reaction. Chinese patent CN102174619A (application number 201110005088.2) disclosed a method for the synthesis of salidroside or analogs prepared by glucose glycosyltransferase. The glucose glycosyltransferase in this patent was derived from *Enterococcus faecium.* Chinese patent CN107937457A (application number 201711141952.5) disclosed a method for the enzymatic catalysis of n-butyl-β-D-glucoside in transglycosylation reaction to synthesize salidroside. The β-glucosidases used in this patent document were β-glucosidase from almonds, β-glucosidase from *Agrobacterium* sp., β-glucosidase from *Phanerochaete chrysosporium,* or β-glucosidase from *Thermotoga maritima.* All salidroside prepared by these above enzymatic methods were β-salidroside.

## SUMMARY

Objective of the Invention: To provide an α-salidroside and a method for preparing α-salidroside, or to provide a process for industrial production of α-salidroside.

Driven by the need for product iteration, the applicant conducted the research on salidroside and unexpectedly discovered that the synthesized glycoside differs from the existing β-salidroside, identified as α-salidroside, an isomer of β-salidroside. In addition, the present invention also provides a method for preparing the α-salidroside and applications thereof.

The prior art has not yet reported on the isomer α-salidroside, let alone studies on the synthesis methods and biological activity of α-salidroside.

### Technical Solution

In one aspect, the present invention provides an α-salidroside, having a structural formula as shown in Formula I, which has an α-glycosidic bond on the alcohol hydroxyl group of tyrosol:

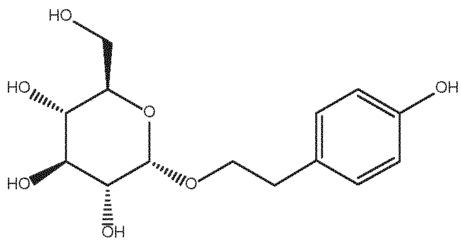

I

According to a preferred embodiment of the present invention, the α-salidroside, upon analysis by LC-MS (liquid chromatography mass spectrometry), has a molecular weight of 300.23.

According to a preferred embodiment of the present invention, the H-NMR spectrum of the α-salidroside is: $^1$H NMR (600MHz, DMSO): δ 2.74 (t, 2H), 3.05-3.06 (m, 1H), 3.19 (m, 1H), 3.31 (m, 2H), 3.40-3.50 (m, 3H), 3.71-3.72 (m, 1H), 4.42-4.35 (m, 1H), 4.59-4.60 (m, 1H), 4.68 (d, J=3.6Hz, 1H: β-H), 4.74 (m, 1H), 4.73-4.84 (m, 1H), 6.67 (d, J=7.8Hz, 2H: H-2, H-6), 7.04 (d, *J*=7.8Hz, 2H: H-3, H-5), 9.1 (s, 1H: OH).

The C-NMR spectrum of the α-salidroside is: $^{13}$C NMR (151MHz, DMSO): δ 35.2, 61.4, 68.8, 70.7, 72.4, 73.3, 73.7, 99.0, 115.5, 129.3, 130.2, 156.0.

The α-salidroside of the present invention is an isomer of the natural β-salidroside, with a chemical name of 2-(4-hydroxyphenyl) ethyl-α-D-glucopyranoside.

The α-salidroside of the present invention has superior antioxidant and free radical scavenging abilities compared to β-salidroside (natural salidroside).

The present invention further provides an application of α-salidroside in the preparation of products with a function of scavenging free radicals.

In the application of α-salidroside in the preparation of products with the function of scavenging free radicals, it is preferred that the free radicals include DPPH radicals and hydroxyl radicals.

In the application of α-salidroside in the preparation of products with the function of scavenging free radicals,

it is further preferred that the products are cosmetics with free radical scavenging capabilities.

In the application of α-salidroside in the preparation of products with the function of scavenging free radicals, it is further preferred that the products are anti-fatigue health supplements.

The present invention further provides a composition containing α-salidroside. The composition has a function of scavenging free radicals, and the free radicals include DPPH radicals and hydroxyl radicals.

The composition is a cosmetic composition containing α-salidroside, with categories selected from aqueous formulations, oily formulations, emulsions, gel products, cream products, or others. It can be produced according to existing cosmetic technology standards without special requirements.

The composition is a health supplement composition containing α-salidroside, which can be in the form of capsules, tablets, creams, liquids, or others. It can be produced according to existing health supplement technology standards without special requirements.

The present invention further provides a method for preparing α-salidroside, which involves dissolving tyrosol and glycosyl donor in a buffer solution, then adding glycosyltransferase, and conducting a glycosylation reaction in the reaction system.

In the present invention, the glycosyltransferase employed exhibits strict specificity and chiral catalytic abilities, enabling it to specifically synthesize α-salidroside, which is different from the glycosyltransferases utilized in existing technologies for the synthesis of β-salidroside.

In a preferred embodiment of the present invention, the glycosyl donor is selected from one or more of maltose, maltotriose, glucose, fructose, starch, soluble starch, and dextrin.

In a preferred embodiment of the present invention, within the reaction system, a concentration of tyrosol is 5 to 400 mg/mL, more preferably is 5 to 100 mg/mL; a concentration of the glycosyl donor is 0.5 to 30 times that of the concentration of tyrosol, more preferably is 2 to 10 times that of the concentration of tyrosol.

In a preferred embodiment of the present invention, the buffer solution is phosphate buffer, acetate buffer, or Good's buffers, with a concentration ranging from 0.01M to 0.5M and a pH value from 5 to 10.

In a preferred embodiment of the present invention, the glycosyltransferase is a commercial glycosyltransferase preparation available on the market or a crude enzyme solution of glycosyltransferase obtained after culturing and purifying microorganisms.

More preferably, the commercial glycosyltransferase preparations available on the market are sourced from Amano Enzyme Co., Ltd. and Novozymes A/S, including enzymes like L-glycosyltransferase, amylase, aromatase, cellulase, cyclodextrin glycosyltransferase, and glycosyltransferase among others, all capable of generating or transferring α-glycosides.

More preferably, the microorganisms include species from genera such as *Arthrobacter sp., Aspergillus sp., Paenibacillus sp., Geobacillus sp., Thermoanaerobacter sp., Aerribacillus sp., Trichoderma sp., Bacillus sp.,* or *Penicillium sp.* More preferably, the microorganisms are species from the genera *Aspergillus sp.* and *Arthrobacter sp.*

In a further preferred embodiment, the microorganism is *Aspergillus niger* and *Arthrobacter sp.* M-238.

As preferred in the present invention, the reaction system may also include lysis co-solvents such as dimethyl sulfoxide (DMSO) or dimethylacetamide (DMA) that do not affect glycosylation reactions, or surfactants such as Tween-20 or Span that do not affect enzymatic reactions.

As preferred in the present invention, the glycosylation reaction is conducted at a temperature ranging from 15 to 50 °C, more preferably ranging from 30 to 50 °C; the glycosylation reaction has a duration of 1 to 100 hours, more preferably 10 to 50 hours.

In the present invention, the preparation of α-salidroside can also be carried out in organic solvents. The organic solvents used in the reaction can be of any type and concentration range that does not interfere with the reaction. These solvents include, but are not limited to, methanol, DMSO, 2-propanol, ethanol, either as a single solvent or a mixture of two or more solvents. The pH of the reaction system is adjusted using the aforementioned buffer solution to be within the range of 5 to 10.

The present invention further provides a method for scavenging free radicals, which comprises administering an effective dose of α-salidroside or a composition containing α-salidroside to a subject. The free radicals include DPPH radicals and hydroxyl radicals.

The term "subject" refers to animals that have undergone treatment, observation, or experimentation. Preferably, it refers to mammals, and most preferably, it refers to humans.

The term "effective dose" refers to the quantity of active compounds or compositions or medicines, including α-rhodiola glycoside, which can induce a biological or medical response in tissue systems, animals, or humans, as sought by researchers, veterinarians, doctors, or other medical professionals. This response includes the clearance or partial clearance of free radicals.

**Beneficial Effects:**

The present invention provides a novel salidroside, namely α-salidroside, which possesses an α-glycosidic bond on the alcohol hydroxyl group of tyrosol. Compared to the known β-salidroside, α-salidroside exhibits superior activity in scavenging DPPH radicals and hydroxyl radicals. It can be used as a new functional ingredient in cosmetics or anti-fatigue health supplements. Further research is performing to explore the new functions of α-salidroside.

The present invention also provides a method for the preparation of α-salidroside. When the glycosyltransferase derived from *Aspergillus* sp. was used, a conversion rate of tyrosol to α-salidroside was above 2%. When the glycosyltransferase derived from *Arthrobacter sp.* was tested, a conversion rate of tyrosol to α-salidroside was 20%. The conversion rate of the glycosylation reaction was significantly increased for industrial production.

The inexpensive glycosyl donors such as starch and tyrosol are used as substrates in the method for preparing α-salidroside according to the present invention. Under the action of glycosyltransferase, α-salidroside is synthesized, and this method is suitable for low-cost industrial production.

**BRIEF DESCRIPTION OF DRAWINGS**

FIG. 1 shows the TLC (Thin Layer Chromatography) of the samples obtained after separating and purifying the enzyme-catalyzed reaction solution from Example 1 in Example 10. In the figure, from left to right are TLC plate 1, and TLC plate 2; in TLC plate 1, from left to right are lane 1, and lane 2.

FIG. 2 is the HPLC (High-Performance Liquid Chromatogram) of the β-salidroside standard.

FIG. 3 is the HPLC of the tyrosol standard.

FIG. 4 shows the HPLC of the samples obtained after separating and purifying the enzyme-catalyzed reaction solution from Example 1 in Example 10.

FIG. 5 shows the LC-MS (Liquid Chromatography-Mass Spectrometry) of the substance at rt (retention time) =10.16 min in the HPLC of the samples obtained after separating and purifying the enzyme-catalyzed reaction solution from Example 1 in Example 10.

FIG. 6 is the LC-MS of the β-salidroside standard.

FIG. 7 shows the H-NMR (proton nuclear magnetic resonance) chart of the substance at rt=10.16 min in the HPLC chromatogram of the samples obtained after separating and purifying the enzyme-catalyzed reaction solution from Example 1 in Example 10.

FIG. 8 shows the C-NMR (carbon nuclear magnetic resonance) chart of the substance at rt=10.16 min in the HPLC chromatogram of the samples obtained after separating and purifying the enzyme-catalyzed reaction solution from Example 1 in Example 10.

**DETAILED DESCRIPTION**

The present invention will now be further described with reference to specific examples. The examples provided are not intended to limit the present invention, and the scope of the present invention is not limited thereto.

The α-salidroside of the present invention is an isomer of natural β-salidroside, with a chemical name of 2-(4-hydroxyphenyl) ethyl-α-D-glucopyranoside. The structure is as follows:

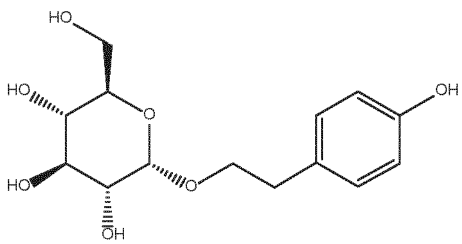

**I.**

In the preparing method for α-salidroside described in the present invention, tyrosol is used as a substrate, and glycosylation is achieved under the action of glucosyltransferase sourced from microbes or commercial glucosyltransferase.

The glucosyltransferase utilizes inexpensive sugar sources such as starch, maltodextrin, and maltose, among

others, for glycosylation of α-linkages.

Commercially available glycosyltransferases which catalyze to form α-glycosidic bond, are obtained from Amano Enzyme Co., Ltd. and Novozymes A/S, and these are like L-glycosyltransferase, amylase, aromatase, cellulase, cyclodextrin glycosyltransferase, and glycosyltransferase among others.

The glycosyltransferases can be used in a form of powder or liquid, or enzymes immobilized on resins can be used to the reaction system. The immobilized enzymes can be reused.

Commercially available enzymes can be utilized or the required enzymes can be produced through microbial cultivation as the fresh enzyme solutions for glycosylation modification.

The microbial sources of glycosyltransferase are such as *Arthrobacter sp., Aspergillus sp., Paenibacillus sp., Geobacillus sp., Thermoanaerobacter sp., Aerribacillus sp., Trichoderma sp., Bacillus sp.,* or *Penicillium sp.,* without particular limitation. The glycosyltransferase can be obtained from microbial cultures through separation and purification, including compositions of glycosyltransferase, purified glycosyltransferase, compositions containing α-glycosyltransferase, purified α-glycosyltransferase, or immobilized α-glycosyltransferase bound to a carrier.

Preparation of enzyme solution: the method of separating and purifying glycosyltransferase from microbial cultures involves using ultrasound or glass beads to disrupt the microbial cells, followed by enzyme purification treatment of the disrupted cell debris or supernatant. The treatment methods include sulfate precipitation, ion exchange column chromatography, chelating affinity chromatography, or gel filtration column chromatography, among others, as well as a combination of aforementioned methods.

Enzyme Immobilization: the carriers for immobilizing microorganisms and the aforementioned glycosyltransferase are not limited, including inorganic carriers such as diatomaceous earth, gypsum, kaolin, silica gel, molecular sieves, porous glass, activated carbon, calcium carbonate, ceramics, ceramic powder, among others., and organic polymers such as polyvinyl alcohol, polypropylene, acrylamide, carrageenan (carrageen), chitosan, ion exchange resins, hydrophobic adsorption resins, chelating resins, synthetic adsorption resins, among others.

The methods of immobilizing microorganisms, enzymes, etc., on carriers include adsorption, ionic bonding, covalent bonding, and bio-specific binding, among others. The immobilized glycosyltransferase can be reused in batch systems or continuous systems for the production of glycosylated compounds.

Selection of glycosyl donors: the raw materials for glycosylation generally include high molecular weight sugar derivatives such as starch, water-soluble starch, dextrin, among others, as well as low molecular weight sugar derivatives such as maltose, maltotriose, glucose, fructose among others.

Preparation of glycosides: the method of preparing α-salidroside as described in the present invention, involves adding glycosyltransferase or microbial cultures to a mixture of tyrosol and glycosyl donors, and carrying out the glycosyltransferase reaction. Lysis co-solvents that do not affect the enzymatic reaction, for example, organic solvents like dimethyl sulfoxide (DMSO) or dimethylacetamide (DMA), and others, or surfactants that do not affect the enzymatic reaction, for example, reagents like Tween-20 and Span, and others, can be used to the enzyme reaction.

When preparing α-salidroside using the aforementioned glucosyltransferases, the amount of glucosyltransferase and reaction conditions greatly impact the production efficiency. Therefore, selecting suitable enzyme quantities and reaction times and other reaction conditions is crucial. From an economic standpoint, the enzyme quantity added in the reaction should be controlled such that the reaction completes in about 1 to 100 hours. Additionally, to ensure full catalysis of substrates by glycosyltransferase, it's preferred that the reaction system has a pH of 5 to 10, and a reaction temperature of 15 to 50°C. When using buffer solutions to adjust the pH, phosphate buffer, acetate buffer, or Good's buffers, and others, can be used at a concentration ranging from 0.01 M to 0.5 M.

In the method for preparing α-salidroside of the present invention, a concentration of tyrosol in the enzymatic reaction can typically range from 5 to 400 mg/mL, with a preferred concentration ranging from 20 to 300 mg/mL. A concentration of glycosyl donor, relative to the substrate tyrosol, is preferably within a range of 0.5 to 30 times.

The reaction between tyrosol and glycosyl donor can be conducted in the presence of solvents. The solvents used in the reaction can be of any type and concentration range as long as they do not affect the reaction, specifically including common reagents such as methanol, DMSO, 2-propanol, ethanol, etc. These solvents can be used individually or in combination of two or more.

The glycosylation reaction in the present invention can be carried out in the presence of a glycosyl donor by contacting microbial cultures. "Contacting microbial cultures" refers to the action of adding tyrosol and glycosyl donor to a system containing microbial cultures for reaction, for instance, allowing a solution of tyrosol and glycosyl donor to flow and contact immobilized microorganisms, completing the glycosylation of tyrosol. In this situation, microorganisms can be present or absent in the culture medium. As mentioned above, "microbial cultures" include the dried cells of microorganisms and their cell extracts, free glycosyltransferases, and immobilized glycosyltransferases. When the culture medium contains sugars such as glucose, fructose, sucrose, mannose, maltose, mannitol, xylose, galactose, starch, dextrin, molasses, sorbitol, glycerol, etc., these sugars are able to serve as glycosyl donors.

The enzyme used in the present invention can be inactivated by methods such as heating or altering the pH value, thereby stopping the enzyme-catalyzed reaction.

Due to the different solubilities of the substrate tyrosol and the glycosylated compounds in water, separation can generally be carried out by extraction. The tyrosol glycosylated compounds are soluble in solvents with higher hydrophilicity, and this hydrophilic solution can be further separated and purified through methods like ion exchange column chromatography, gel filtration chromatography, or hydrophobic chromatography, among others, making it easy to obtain high-purity refined glycoside products.

The aforementioned glycoside mixtures, hydrolysis products of the mixtures, enzyme inactivation products of the mixtures, and purified products of the mixtures, in their dried powder form, can be used in glycoside-containing food, beverages, and cosmetics. They can also apply as ingredients in food, special medical food, health supplements, or pharmaceuticals, for use in food and beverages, cosmetics, special medical food, health supplements, or pharmaceuticals.

TLC analysis conditions:

Tyrosol, being the substrate for the enzyme-catalyzed reaction, contains a phenyl ring in its molecular structure, hence it has ultraviolet absorption and can be detected at a wavelength of 254 nm using a UV detector. Thin Layer Chromatography serves as a simple and convenient method for analyzing the enzyme reaction solution and has been employed for studying the separation conditions.

Purchasing commercial high-purity tyrosol and β-salidroside (natural salidroside) as standard substances for the analysis or identification of products (hereinafter referred to as: tyrosol standard, β-salidroside standard), using commercially available TLC plates from Merck (Merck Kieselgel 60 F254), with a solution of chloroform : methanol : acetic acid = 5:1:1 (volume ratio) as the developing solvent, tyrosol standard: Rf=0.9, β-salidroside standard: Rf=0.4.

HPLC analysis conditions:

High Performance Liquid Chromatography: Hitachi HPLC 5440 chromatograph, Chromatographic column: Unison UK-C18 (150×4.6mm, 3μm), Detector: Photodiode Array (DAD 280nm), Detection wavelength: 280nm, Injection volume: 10μL, Flow rate: 0.5mL/min, Column temperature: 30°C, Mobile phase: Acetonitrile: 0.1% formic acid aqueous solution gradient elution, elution gradient (v/v) as shown in Table 1.

Table 1: Gradient Elution Table

| Analytical Time, min | Acetonitrile | 0.1% Formic Acid Aqueous Solution (v/v) |
|---|---|---|
| 0-10 | 10%-20% | 90%-80% |
| 10-15 | 20%-21% | 80%-79% |
| 15-20 | 21%-95% | 79%-5% |
| 20-35 | 95%-10% | 5%-90% |

LC-MS analysis conditions:

Column: Unison UK-C18 (150x4.6mm, 3μm), Detector: Photodiode Array (DAD 280nm), Detection Wavelength: 280nm, Injection Volume: 10μL, Flow Rate: 0.5mL/min, Column Temperature: 30°C, Mobile Phase as per Table 1 for detection time 10-15min; H-ESI mode, Molecular Weight Scanning Range: 50-800.

**Example 1. Glycosylation Experiment with L-Glycosyltransferase**

10 mg of tyrosol (molecular weight 138.16), 20 mg of soluble starch, and 20 mg of maltose were dissolved in 0.9 mL of 0.2 M sodium acetate buffer (pH 5.6). The reaction mixture was stirred and then added into120 μL of enzyme solution of L-glycosyltransferase (TG-L, Amano Enzyme Co., Ltd.) sourced from *Aspergillus niger.* The total mixture was stirred at 50°C overnight.

**Example 2. Glycosylation Experiment with Cellulase A**

10 mg of tyrosol (molecular weight 138.16), 20 mg of soluble starch, and 20 mg of maltose were dissolved in 0.9 mL of 0.2 M sodium acetate buffer (pH 5.6). The reaction mixture was stirred and then added into 120 μL of enzyme solution of Cellulase A (cellulase A, Amano Enzyme Co., Ltd.) sourced from *Aspergillus niger.* The total mixture was stirred at 50°C overnight.

**Example 3. Glycosylation Experiment with Cyclodextrin Glycosyltransferase (CGTase)**

10 mg of tyrosol (molecular weight 138.16) and 60 mg of soluble starch were dissolved in 0.8 mL of 0.2 M sodium acetate buffer (pH 5.6). The reaction mixture was stirred and then added into 120 μL of enzyme solution of Cyclodextrin Glycosyltransferase (CGTase, Contizyme, from Amano Enzyme Co., Ltd.) sourced from *Paenibacillusmacerans.* The total mixture was stirred at 50°C overnight.

**Example 4. Glycosylation Experiment with Cyclodextrin Glycosyltransferase (CGT-SL)**

10 mg of tyrosol (molecular weight 138.16) and 60 mg of soluble starch were dissolved in 0.8 mL of 0.2 M sodium acetate buffer (pH 5.6). The reaction mixture was stirred and then added into 120 μL of enzyme solution of Cyclodextrin Glycosyltransferase (CGT-SL, from Amano Enzyme Co., Ltd.) sourced from *Geobacillus sp.* The total mixture was stirred at 50°C overnight.

**Example 5. Glycosylation Experiment with Cyclodextrin Glycosyltransferase (Toruzyme)**

10 mg of tyrosol (molecular weight 138.16) and 60 mg of soluble starch were dissolved in 0.8 mL of 0.2 M sodium acetate buffer (pH 5.6). The reaction mixture was stirred and then added into 120 μL of enzyme solution of Cyclodextrin Glycosyltransferase (Toruzyme, from Novozymes A/S) sourced from *Thermoanaerobacter sp.* The total mixture was stirred at 50°C overnight.

**Example 6. Glycosylation Experiment with Glycosyltransferase (Glucose transferase-L)**

10 mg of tyrosol (molecular weight 138.16), 20 mg of soluble starch, 20 mg of maltose were dissolved in 0.9 mL of 0.2 M sodium acetate buffer (pH 5.6). The reaction mixture was stirred and then added into 120 μL of enzyme solution of glycosyltransferase (Glucose transferase-L, Amano Enzyme Co., Ltd.) sourced from *Aerribacillus sp.* The mixture was stirred at 50°C overnight.

**Example 7. Glycosylation Experiment with Cellulase T**

10 mg of tyrosol (molecular weight 138.16), 20 mg of soluble starch, and 20 mg of maltose were dissolved in 0.9 mL of 0.2 M sodium acetate buffer (pH 5.6). The reaction mixture was stirred and then added into 120 μL of enzyme solution of cellulase (cellulase T, Amano Enzyme Co., Ltd.) sourced from *Trichoderma viride.* The total reaction mixture was stirred at 50°C overnight.

**Example 8. Glycosylation Experiment with β-Amylase**

10 mg of tyrosol (molecular weight 138.16), 20 mg of soluble starch, and 20 mg of maltose were dissolved in 0.9 mL of 0.2 M sodium acetate buffer (pH 5.6). The reaction mixture was stirred and then added into 120 μL of enzyme solution of amylase (β-amylase F, Amano Enzyme Co., Ltd.) sourced from *Bacillus flexus.* The total reaction mixture was stirred at 50°C overnight.

**Example 9. Glycosylation Experiment with Aromatase**

10 mg of tyrosol (molecular weight 138.16), 20 mg of soluble starch, and 20 mg of maltose were dissolved in 0.9 mL of 0.2M sodium acetate buffer (pH 5.6). The reaction mixture was stirred and then added into 120 μL of enzyme solution of aromatase (Aromase, Amano Enzyme Co., Ltd.) sourced from *Penicillium multicolor.* The total reaction mixture was stirred at 50°C overnight.

**Example 10: Preparation, Purification, and Structural Analysis of α-salidroside**

The enzyme reaction solution obtained from Example 1 was separated and purified, and then analyzed using TLC, HPLC, LC-MS, and NMR.

(1) Separation and Purification

A hydrophobic polymer resin (HP-20, macroporous adsorption resin, Solarbio brand) with a 5% ethanol solution was used to separate and purify the reaction solution. Subsequently, a silica gel resin (ODS) with a 40% ethanol solution

was used for further separation and purification, obtaining a high-purity enzyme-catalyzed reaction solution.

(2) TLC Analysis

Utilizing TLC plates produced by Merck (Merck Kieselgel 60 F254), and employing a solvent system of chloroform: methanol: acetic acid = 5:1:1 for development, tyrosol (standard, lane 1) and salidroside (β-salidroside standard, lane 2) were developed on TLC plate 1, while the enzyme-catalyzed reaction solution obtained from Example 1 was developed on TLC plate 2, followed by TLC analysis. Some of spots on the TLC plates were detected under a UV lamp. Since the Rf value of the tyrosol standard in TLC plate 1 is 0.9, and the Rf value of β-salidroside standard is 0.4, a spot near the Rf value of 0.4 on TLC plate 2 was identified, suggesting the synthesis of a β-salidroside analog in the enzymatic reaction. Refer to FIG. 1 for details.

(3) HPLC Analysis

The HPLC analysis was performed using a Hitachi HPLC 5440 chromatograph, with a C18 column (Unison UK-C18, 150 mm × 4.6 mm, 3 μm) at a column temperature of 30°C. The mobile phase was a gradient elution of acetonitrile: 0.1% formic acid aqueous solution (v/v), utilizing a photodiode array detector at UV 280 nm.

In accordance with the aforementioned chromatographic conditions, the β-salidroside standard was analyzed through HPLC, with the HPLC chromatogram detailed in FIG. 2.

In accordance with the aforementioned chromatographic conditions, the tyrosol standard was analyzed through HPLC, with the HPLC chromatogram detailed in FIG. 3.

In accordance with the aforementioned chromatographic conditions, the product obtained after the separation and purification of the enzyme-catalyzed reaction solution from Example 1 was analyzed through HPLC, with the HPLC chromatogram detailed in FIG. 4.

The HPLC chromatograms in FIGs. 2, 3, and 4 showed that the retention time (rt) for the β-salidroside standard is 9.72 min, while the retention time for the tyrosol standard is 12.78 min. The enzyme-catalyzed solution from Example 1 showed two peaks, with retention times of 10.16 min and 12.83 min, respectively. The peak at rt=12.83 min corresponds to tyrosol, and the peak at rt=10.16 min exhibits a noticeable difference in retention time compared with β-salidroside standard.

(4) LC-MS Analysis

In this step, the substance with a retention time of 10.16 min was analyzed through LC-MS. The measured value for the molecular ion peak [M-1] was found to be 299.11. The results are detailed in FIG. 5.

The HPLC retention time of salidroside standard (formula: $C_{14}H_{20}O_7$, molecular weight: 300.23) was 9.72 min. By analyzing through LC-MS, the molecular ion peak of [M-1] with a measured value of 299.11, identifying it as a $[M-H]^+$ peak of Tyrosol glycoside (molecular weight 300), as shown in FIG. 6. This indicated that the substance at the retention time of 10.16 min is a glycoside with a molecular weight of 300.23. Based on the UV spectrogram shown by the photodiode array detector and the LC-MS spectrum, the new peak at retention time rt=10.16 min in the HPLC spectrum was identified as new Tyrosol glycoside. As it has the same molecular weight as a monosaccharide glycoside, a preliminary analysis suggests that this substance is a glycoside formed by the combination of glucose and tyrosol.

(5) H-NMR and C-NMR Spectrum Analysis

The substance at rt=10.16 min was analyzed through H-NMR shown in FIG. 7 and C-NMR spectrum shown in FIG. 8.

The H-NMR spectrum of the product from Example 1 is: $^1$H NMR (600MHz, DMSO): δ 2.74 (t, 2H), 3.05-3.06 (m, 1H), 3.19 (m, 1H), 3.31 (m, 2H), 3.40-3.50 (m, 3H), 3.71-3.72 (m, 1H), 4.42-4.35 (m, 1H), 4.59-4.60 (m, 1H), 4.68 (d, *J*=3.6Hz, 1H: β-H), 4.74 (m, 1H), 4.73-4.84 (m, 1H), 6.67 (d, *J*=7.8Hz, 2H: H-2, H-6), 7.04 (d, J=7.8Hz, 2H: H-3, H-5), 9.1 (s, 1H: OH).

The C-NMR spectrum of the product from Example 1: $^{13}$C NMR (151MHz, DMSO): δ 35.2, 61.4, 68.8, 70.7, 72.4, 73.3, 73.7, 99.0, 115.5, 129.3, 130.2, 156.0.

Based on the H-NMR and C-NMR spectrum of the product from Example 1, along with the nuclear magnetic data of salidroside standard (β-salidroside), the H-NMR spectrum of the product from Example 1 at 4.68 ppm, with a coupling constant of 3.6 Hz, (d, J=3.6Hz, 1H: β-H), represents "the characteristic of an α-glycosidic linkage". The salidroside standard (β-salidroside) has two peaks at 4.29 ppm with a coupling constant of 8.0 Hz, which is the characteristic of β-salidroside, showing a clear distinction between the two analogs. Combining with the literature reference (Bassanini I, J Krejzová, Panzeri W, et al. A Sustainable One-Pot, Two-Enzyme Synthesis of Naturally Occurring Arylalkyl Glucosides[J]. Chemsuschem, 2017,10, 2040-2045. doi:10.1002/cssc.201700136), it's confirmed that the product of Example 1 is α-glucoside. From the TLC analysis, both are the same spot; from the LC-MS analysis, they have the same molecular weight; from the HPLC analysis, they are two different substances. Further, combining with the α-linkage feature in the NMR spectrum (different from the natural β-linkage), it's confirmed that this substance is α-salidroside, which is a stereoisomer of β-salidroside (natural salidroside). Its structural formula is shown as Formula I:

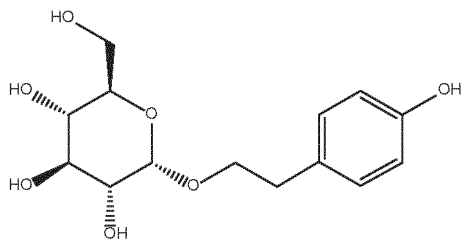

I

(6) The enzyme-catalyzed reaction solutions obtained from Examples 2 to 9 were separately purified, and analyzed using TLC, HPLC, LC-MS, and NMR, and the results were consistent with data from Example 1.

Through HPLC detection, in Examples 1 and 2, the conversion rate of tyrosol to α-salidroside was found to be 2% and 3%, respectively. For the enzyme-catalyzed reaction solutions obtained from Examples 3 to 9, α-salidroside could be detected, but the content was very low.

**Example 11. Glycosylation using microbial cultures**

Step 1. Preparation of Agar Medium

Maltose (Nippon ShokuhinKako, Japan) 5 g, yeast extract (Becton Dickinson) 100mg, ammonium sulfate (Wako Pure Chemical Industries, Japan) 2 g, potassium dihydrogen phosphate (Kanto Chemical Co., Inc., Japan) 1 g, dipotassium hydrogen phosphate (Wako Pure Chemical Industries, Japan) 1 g, magnesium sulfate heptahydrate (Kanto Chemical Co., Inc., Japan) 0.2 g, ferrous sulfate heptahydrate (Kanto Chemical Co., Inc., Japan) 0.01 g, along with 15 g of agar BA-10 (Ina Food Industry Co., Ltd.) were transferred into a 2 L beaker, and 1 L of distilled water was added to dissolve the ingredients. The pH was adjusted to 7.0 using NaOH. The solution was then sterilized in an autoclave at 121°C for 20 minutes. Subsequently, it was poured onto petri dishes on a clean workbench, with 15 mL per dish. The petri dish lids were gently opened, and the medium was allowed to cool and solidify for 20 minutes, to be used as the agar medium.

Step 2. Preparation of Liquid Medium

Maltose (Nippon ShokuhinKako, Japan) 50 g, polypeptone (Wako Pure Chemical Industries, Japan) 2 g, yeast extract (Becton Dickinson) 100 mg, ammonium sulfate (Wako Pure Chemical Industries, Japan) 2 g, potassium dihydrogen phosphate (Kanto Chemical Co., Inc., Japan) 1 g, and dipotassium hydrogen phosphate (Wako Pure Chemical Industries, Japan) 1 g, were added into a 2 L beaker, followed by the addition of 1 L distilled water to dissolve the ingredients. The pH was adjusted to 7.0 using NaOH. Then, 50 mL of the solution was transferred into a 200 mL conical flask, which was then sterilized in an autoclave at 121°C for 20 minutes. After cooling, it was used as the liquid medium.

Step 3. Preparation of Crude Enzyme Solution

*Arthrobacter sp.* M-238 (NITE preservation number AP-02396) which had been cultured on the aforementioned agar medium at 25°C for 2 to 3 days was inoculated into the liquid medium described in Step 2, using 2 inoculating loops, and then incubated at 25°C with shaking at 160 rpm for 42 hours. After incubation, the bacterial cells were removed by centrifugation, and the supernatant was subjected to ammonium sulfate precipitation. Following desalting, glycosyltransferase was fractionally separated using an anion exchange resin, yielding the crude enzyme solution.

Step 4. Glycosylation Reaction

10 mg of tyrosol (molecular weight 138.16) and 20 mg of maltose were dissolved in 1 mL of 0.1 M phosphate buffer (pH 7.0). Then, 20 μL of the aforementioned crude enzyme solution was added to the mixture, and it was stirred at 30°C overnight to obtain the enzyme-catalyzed reaction solution.

Through TLC, HPLC, and LC-MS analyses, the formation of α-salidroside was confirmed. According to HPLC

analysis, the conversion rate of tyrosol to α-salidroside was about 20%.

The above experiments found that when the glycosyltransferase was derived from the genus *Arthrobacter sp.,* the conversion rate of tyrosol to α-salidroside is 20%. Compared to the commercial enzyme preparations mentioned above, the conversion rate of tyrosol to α-salidroside has significantly increased.

Industrial Feasibility:

The present invention utilizes glycosyltransferase, particularly those derived from the genus *Arthrobacter sp.,* to glycosylate tyrosol derivatives. The glycosides formed through glycosylation not only retain the original properties of the tyrosol derivatives but also further enhance their physiological functions, thereby increasing their developmental value. They can be applied as raw materials for cosmetics, health supplements, and pharmaceuticals.

The following example pertains to a free radical scavenging experiment, with materials and reagents as shown in Table 2.

Table 2: Materials and Reagents

| Reagent Name | Reagent Type | Purity | Manufacturer |
|---|---|---|---|
| Anhydrous Ethanol | Analytical Grade | ≥99.7% | Sinopharm Chemical Reagent Co., Ltd |
| DPPH (1,1-Diphenyl-2-picrylhydrazyl) | - | 98% | Shanghai yuanye Bio-Technology Co., Ltd |
| β-salidroside | - | 98% | Shanghai Macklin Biochemical Co., Ltd |
| Ferrous Sulfate Heptahydrate | Analytical Grade | 99~101% | Sinopharm Chemical Reagent Co., Ltd |
| Sodium Dihydrogen Phosphate | Analytical Grade | - | Sinopharm Chemical Reagent Co., Ltd |
| Disodium Hydrogen Phosphate | Analytical Grade | - | Sinopharm Chemical Reagent Co., Ltd |
| 1,10-Phenanthroline | - | 99% | Shanghai yuanye Bio-technology Co., Ltd |
| Hydrogen Peroxide | Analytical Grade | 30% | Sinopharm Chemical Reagent Co., Ltd |

**Experiment 12: DPPH Radical Scavenging Experiment**

Free radicals play a significant role, either directly or indirectly, in oxidative processes and are widely involved in the physiological and pathological processes of the body. When the body has an excess of free radicals, the body damage can be caused by free radicals through oxidative actions. Phenolic compounds, being donors of phenolic hydroxyl groups, form the structural basis for antioxidant activity. This experiment employs the DPPH [1,1-Diphenyl-2-trinitrophenyl hydrazine, also known as 1,1-Diphenyl-2-picrylhydrazyl, (free radical)] radical scavenging reaction to study the scavenging efficiency of α-salidroside and β-salidroside on DPPH radicals.

1. Solution Preparation:

(1) α-salidroside solution: prepared as a 500 mg/L aqueous solution using conventional methods.
(2) β-salidroside solution: prepared as a 500 mg/L aqueous solution using conventional methods.
(3) DPPH ethanol solution: prepared as a 0.04 mg/ml solution in anhydrous ethanol using conventional methods.

2. DPPH Radical Scavenging Experiment:

(1) 0.9 mL each of α-salidroside solution and β-salidroside aqueous solution were taken separately, and 0.9 mL of DPPH anhydrous ethanol solution was added to each, mixed well, and then left to stand in the dark. Samples were taken at 0.5 h, 1 h, 2 h, and 4 h respectively, and the absorbance was measured at a wavelength of 517 nm. The average of three parallels was calculated and noted as Ai.
(2) 0.9 mL each of α-salidroside solution and β-salidroside aqueous solution were taken separately, and 0.9 mL of anhydrous ethanol was added to each, mixed well, and then left to stand in the dark for 1 hour. Samples were taken at 0.5 h, 1 h, 2 h, and 4 h respectively, and the absorbance was measured at a wavelength of 517 nm. The average of three parallels was calculated and noted as Aj.
(3) 0.9 mL of distilled water was taken, to which 0.9 mL of DPPH ethanol solution was added, mixed well, and the absorbance was measured at a wavelength of 517 nm. The average of three parallels was calculated and noted as A4.

(4) 0.9 mL of anhydrous ethanol was taken, to which 0.9 mL of distilled water was added, mixed well, and was used as a blank.

The DPPH radical scavenging rate was calculated according to the formula below, and the results were recorded in Table 3.

$$\text{DPPH Radical Scavenging Rate} = [(A4 - Ai + Aj) / A4] \times 100\%$$

Table 3: DPPH Radical Scavenging Rate

| Sample \ Scavenging Rate | DPPH Radical Scavenging Rate, % | | | |
|---|---|---|---|---|
| | 0.5h | 1h | 2h | 4h |
| β-salidroside | 19.96 | 23.21 | 28.24 | 28.33 |
| α-salidroside | 19.29 | 28.45 | 31.00 | 34.12 |

The data in Table 3 indicates that α-salidroside has a higher capability of scavenging DPPH radicals compared to β-salidroside, especially when the reaction time is over 1 hour.

**Example 13. Hydroxyl Radical Scavenging Experiment**

The Hydroxyl Radical Scavenging Activity is an important indicator for measuring the antioxidant capacity of substances. Currently, the main analytical testing methods for hydroxyl radicals include High Performance Liquid Chromatography, Chemiluminescence, Fluorescence Analysis, and Spectrophotometry, etc. Among them, the most commonly used method for measuring the hydroxyl radical scavenging activity is through Spectrophotometry, which can be used to determines the inhibition of the absorbance decline of the colorant 1,10-Phenanthroline by the sample, thereby reflecting the ability of the sample to scavenge hydroxyl radicals. The measurement principle is as follows: $H_2O_2/Fe^{2+}$ produces hydroxyl radicals through the Fenton reaction, oxidizing $Fe^{2+}$ in the 1,10-Phenanthroline-$Fe^{2+}$ aqueous solution to $Fe^{3+}$, leading to a decline in the absorbance at 536 nm. The degree to which the sample inhibits the rate of absorbance decline at 536 nm reflects the ability of the sample to scavenge hydroxyl radicals.

The degree to which the rate of decrease in absorbance is inhibited reflects the ability of the sample to scavenge hydroxyl radicals.

By measuring the changes in the absorbance value of the indicator, the production of hydroxyl radicals can be indirectly determined.

1. Solution Preparation:

(1) $FeSO_4$ Solution: 1.5 mM and 5 mM $FeSO_4$ aqueous solutions were prepared using conventional methods.
(2) PBS Buffer:

1) 0.2 mol/L sodium dihydrogen phosphate and 0.2 mol/L disodium hydrogen phosphate aqueous solutions were prepared.
2) 19 mL of sodium dihydrogen phosphate aqueous solution and 81 mL of disodium hydrogen phosphate aqueous solution were taken, mixed well, and the pH was adjusted to 7.4 (measured with a pH meter, and the pH value was adjusted with 0.1 mol/L sodium hydroxide or 0.1 mol/L phosphoric acid if necessary).

(3) 1,10-Phenanthroline: 0.75 mM and 3 mM aqueous solutions were prepared using conventional methods.
(4) Hydrogen Peroxide ($H_2O_2$) Solution: 0.1% (v/v) aqueous solution was prepared using conventional methods.
(5) α-salidroside Solution: 1 mg/mL and 4 mg/mL aqueous solutions were prepared using conventional methods.
(6) β-salidroside Solution: 1 mg/mL and 4 mg/mL aqueous solutions were prepared using conventional methods.

2. Hydroxyl Radical Scavenging Experiment:

Scheme 1: Using 5 mM $FeSO_4$ aqueous solution, 0.1% hydrogen peroxide, 1 mg/mL α-salidroside aqueous solution or 1 mg/mL β-salidroside aqueous solution, and 3 mM 1,10-Phenanthroline aqueous solution, the following operation steps were carried out for the reaction.

Scheme 2: Using 1.5 mM $FeSO_4$ aqueous solution, 0.1% hydrogen peroxide, 4 mg/mL α-salidroside aqueous solution or 4 mg/mL β-salidroside aqueous solution, and 0.75 mM 1,10-Phenanthroline aqueous solution, the following operation steps were carried out for the reaction.

Specific Operation Steps: (1) 40 μL of α-salidroside solution or β-salidroside solution was taken separately, to which 40 μL of $FeSO_4$ solution, 60 μL of PBS buffer solution (pH 7.4), and 40 μL of 1,10-Phenanthroline solution were added respectively, mixed well. Then, 40 μL of $H_2O_2$ solution was added and mixed well again. The mixture was reacted at 37°C in the dark for 30 minutes. Using distilled water as blank, the absorbance at 536 nm wavelength was measured and the average of three parallels was calculated and noted as $A_i$.

(2) 80 μL of distilled water was taken, to which 40 μL of $FeSO_4$ solution, 60 μL of PBS buffer solution (pH 7.4), and 40 μL of 1,10-Phenanthroline solution were added and mixed well. The mixture was reacted at 37°C in the dark for 30 minutes. Using distilled water as blank, the absorbance at 536 nm wavelength was measured and the average of three parallels was calculated and noted as $A_j$.

(3) 40 μL of distilled water was taken, to which 40 μL of $FeSO_4$ solution, 60 μL of PBS buffer solution (pH 7.4), and 40 μL of 1,10-Phenanthroline solution were added and mixed well. Then, 40 μL of $H_2O_2$ solution was added and mixed well again. The mixture was reacted at 37°C in the dark for 30 minutes. Using distilled water as blank, the absorbance at 536 nm wavelength was measured and the average of three parallels was calculated and noted as $A_0$.

$$\text{Hydroxyl Radical Scavenging Rate} = [(A_i - A_0)/(A_j - A_0)] \times 100\%$$

The results of hydroxyl radical scavenging were recorded in Table 4.

Table 4: Hydroxyl Radical Scavenging Rate

| Scavenging Rate / Sample | Hydroxyl Radical Scavenging Rate, % | |
|---|---|---|
| | Scheme 1 | Scheme 2 |
| β-salidroside | 13.92 | 4.05 |
| α-salidroside | 28.69 | 21.62 |

The data shown in Table 4 from the two detection methods indicate: the capability of α-salidroside in scavenging hydroxyl radicals is far superior to that of β-salidroside.

Although the invention has been described in considerable detail for clarity of understanding through the description and examples, it will be clear to a person skilled in the art that various changes and modifications can be practiced within the scope of the appended claims. Therefore, the description and examples should not be construed as limiting the scope of the invention.

## Claims

1. An α-salidroside, having a structural formula as shown in Formula I, which has an α-glycosidic bond on the alcohol hydroxyl group of tyrosol:

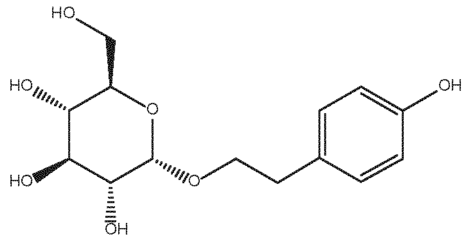

I.

2. An application of the α-salidroside as claimed in claim 1 in the preparation of products with functions of scavenging free radicals;

preferably, the free radicals comprise DPPH radicals and hydroxyl radicals;
preferably, the products are cosmetics with free radical scavenging capabilities;
preferably, the products are anti-fatigue health supplements.

3. A composition containing α-salidroside;

preferably, the composition has the function of scavenging free radicals;
preferably, the free radicals comprise DPPH radicals and hydroxyl radicals;
preferably, the composition is a cosmetic composition, categories of the cosmetic composition are selected from aqueous formulations, oily formulations, emulsions, gel products, and cream products;
preferably, the composition is a health supplement composition, dosage forms of the health supplement composition are selected from capsules, tablets, creams, and liquids.

4. A method for preparing the α-salidroside as claimed in claim 1, comprising: dissolving tyrosol and a glycosyl donor in a buffer solution, then adding a glycosyltransferase to form a reaction system, and conducting a glycosylation reaction in the reaction system.

5. The method as claimed in claim 4, wherein the glycosyl donor is selected from one or more of maltose, maltotriose, glucose, fructose, starch, soluble starch, and dextrin.

6. The method as claimed in claim 4, wherein within the reaction system, a concentration of tyrosol is 5 to 400 mg/mL, preferably is 5 to 100 mg/mL; a concentration of the glycosyl donor is 0.5 to 30 times that of the concentration of tyrosol, preferably is 2 to 10 times that of the concentration of tyrosol;
preferably, the buffer solution is a phosphate buffer, acetate buffer, or Good's buffers, with a concentration ranging from 0.01 M to 0.5 M and a pH value from 5 to 10.

7. The method as claimed in claim 4, wherein the glycosyltransferase is a commercial glycosyltransferase preparation available on the market or a crude enzyme solution of glycosyltransferase obtained after culturing and purifying microorganisms.

8. The method as claimed in claim 7, wherein the commercial glycosyltransferase preparation available on the market is selected from one or more of L-glycosyltransferase, amylase, aromatase, cellulase, cyclodextrin glycosyltransferase, and glycosyltransferase;

preferably, the microorganism is selected from at least one of the following genera: *Arthrobacter sp., Aspergillus sp., Paenibacillus sp., Geobacillus sp., Thermoanaerobacter sp., Aerribacillus sp., Trichoderma sp., Bacillus sp.,* and *Penicillium sp;*
preferably, the microorganisms are species from the *genera Aspergillus sp. and Arthrobacter sp.;*
preferably, the microorganism *is Aspergillus niger or Arthrobacter sp.* M-238.

9. The method as claimed in any one of claims 4 to 8, wherein the reaction system may also comprise lysis co-solvents dimethyl sulfoxide or dimethylacetamide that do not affect glycosylation reactions, or surfactants Tween-20 or Span that do not affect enzymatic reactions;
preferably, the glycosylation reaction is conducted at a temperature ranging from 15 to 50°C, preferably ranging from 30 to 50°C; the glycosylation reaction has a duration of 1 to 100 hours, more preferably 10 to 50 hours.

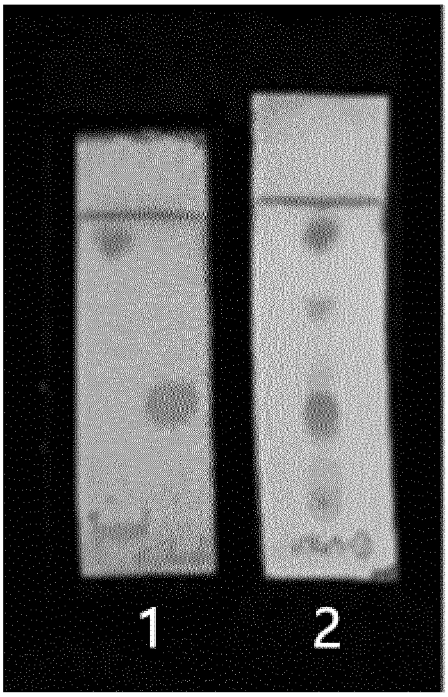


FIG. 1

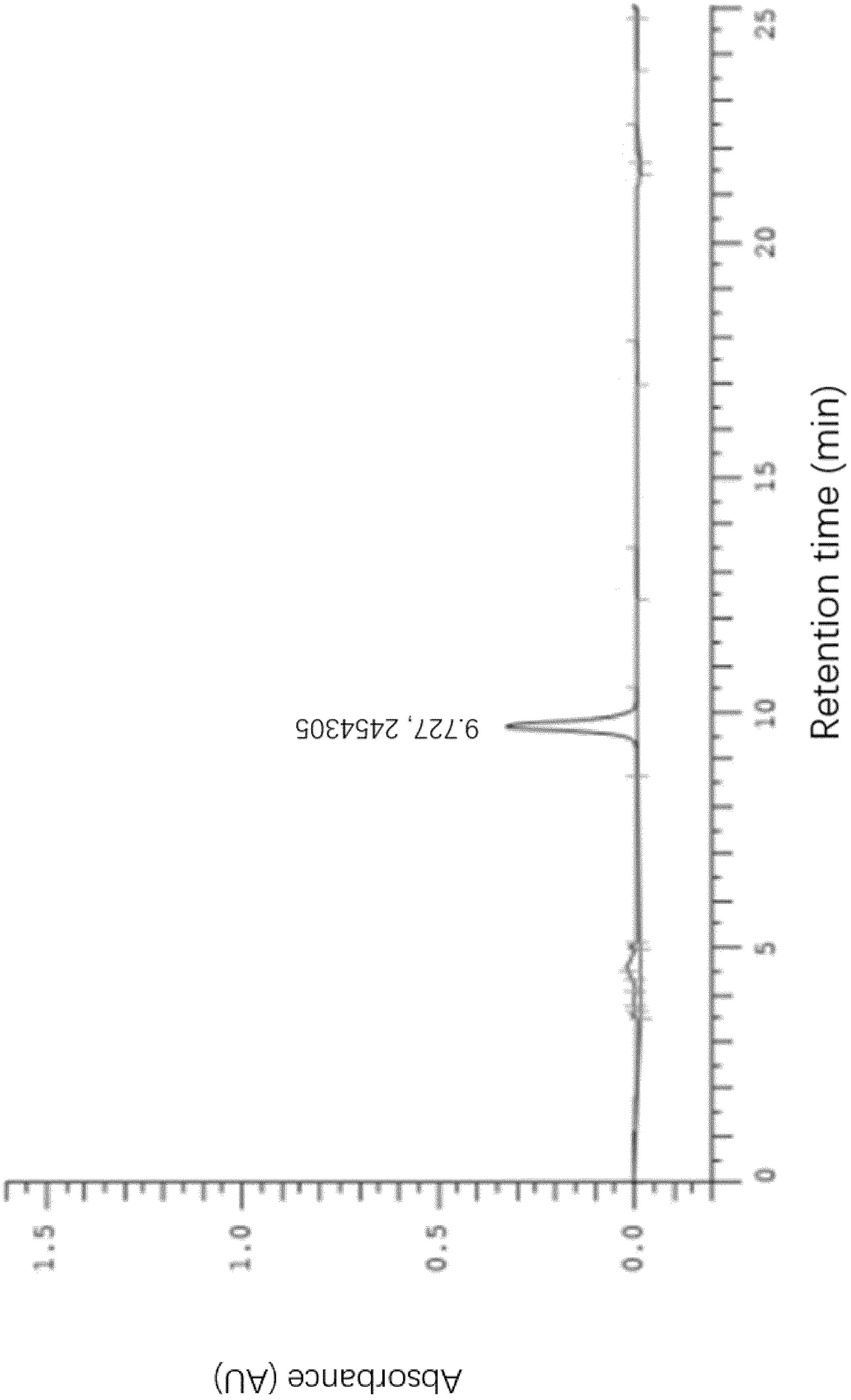
Absorbance (AU)
1.5
1.0
0.5
0.0
Retention time (min)
0
5
10
15
20
25
9.727, 2454305

FIG. 2

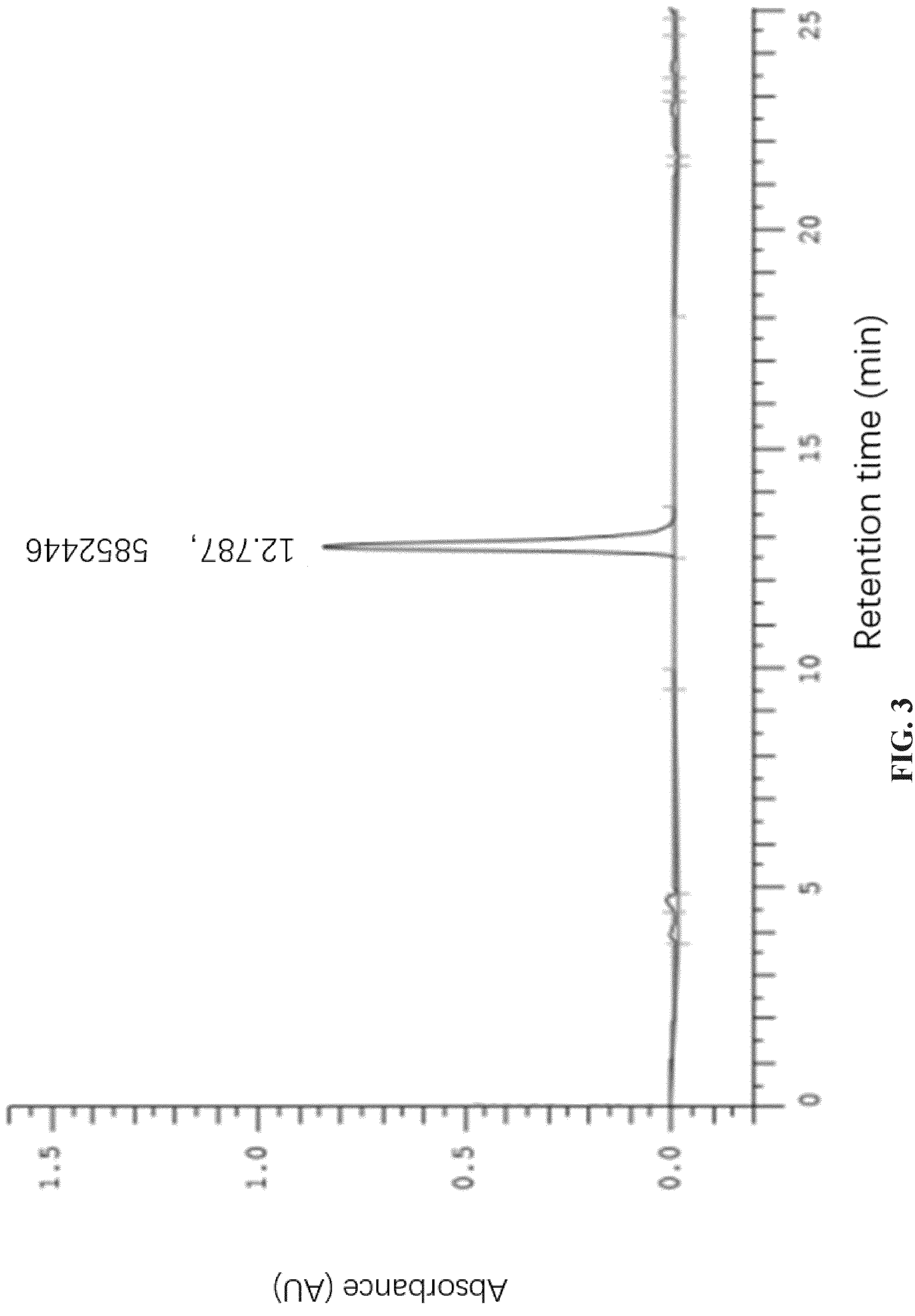
Absorbance (AU)
1.5
1.0
0.5
0.0
Retention time (min)
0
5
10
15
20
25
12.787, 5852446

FIG. 3

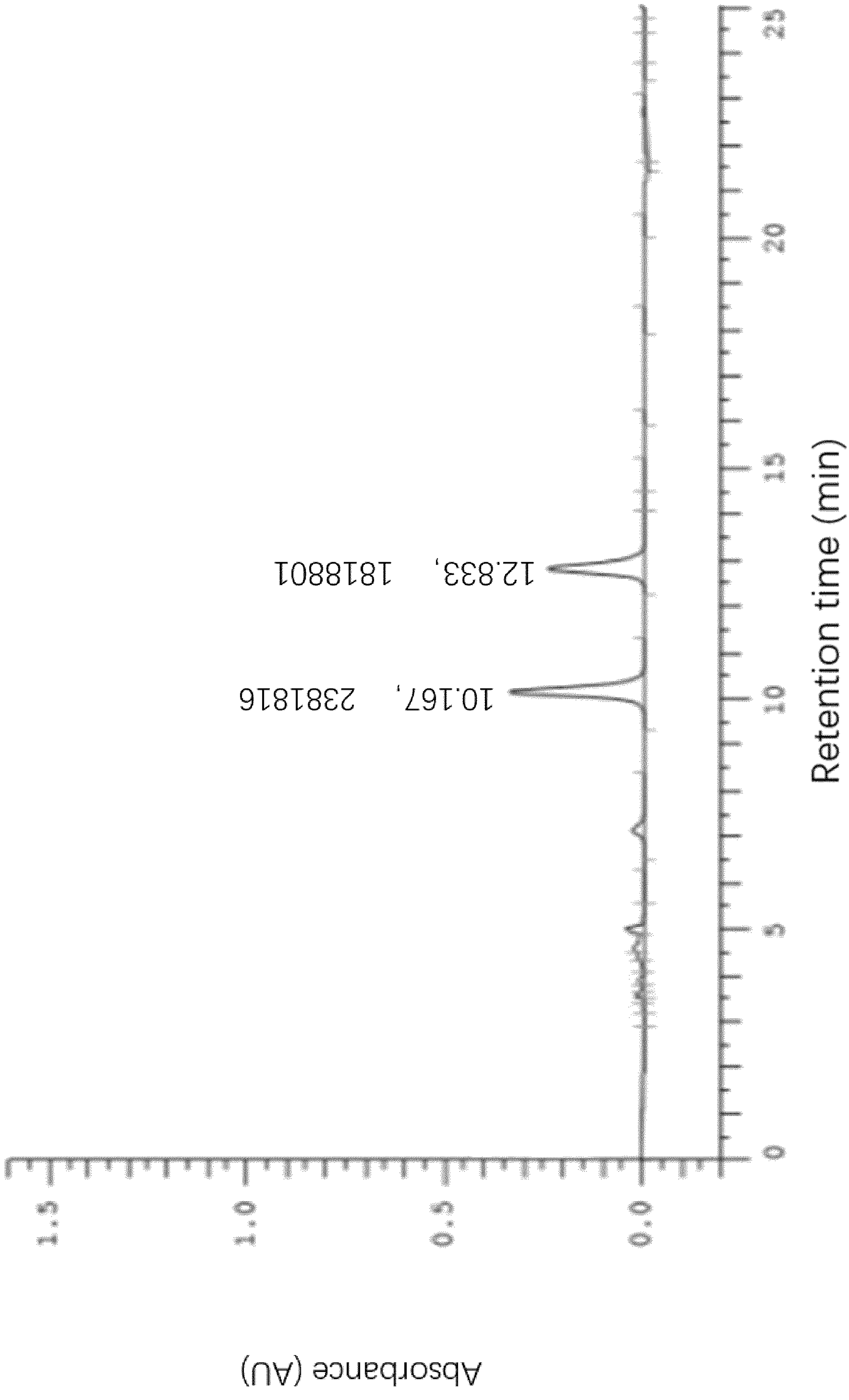
Absorbance (AU)
1.5
1.0
0.5
0.0
Retention time (min)
0
5
10
15
20
25
10.167, 2381816
12.833, 1818801

FIG. 4

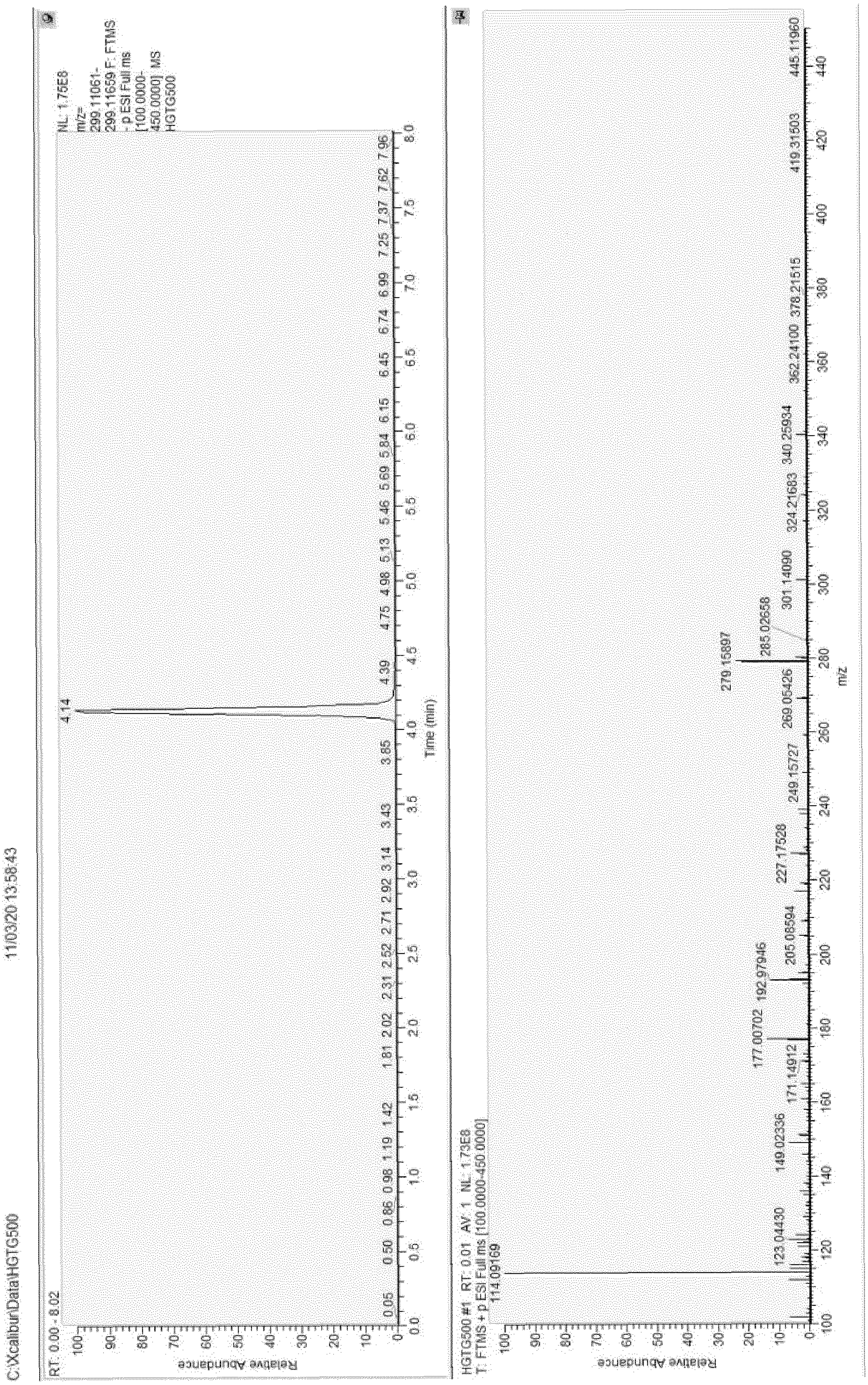
C:\Xcalibur\Data\HGTG500
11/03/20 13:58:43
RT: 0.00 - 8.02
NL: 1.75E8
m/z= 299.11061-299.11659 F: FTMS - p ESI Full ms [100.0000-450.0000] MS HGTG500
Relative Abundance
Time (min)
4.14
HGTG500 #1 RT: 0.01 AV: 1 NL: 1.73E8
T: FTMS + p ESI Full ms [100.0000-450.0000]
114.09169
123.04430
149.02336
171.14912
177.00702
192.97946
205.08594
227.17528
249.15727
269.05426
279.15897
285.02658
301.14090
324.21683
340.25934
362.24100
378.21515
419.31503
445.11960
m/z

FIG. 5

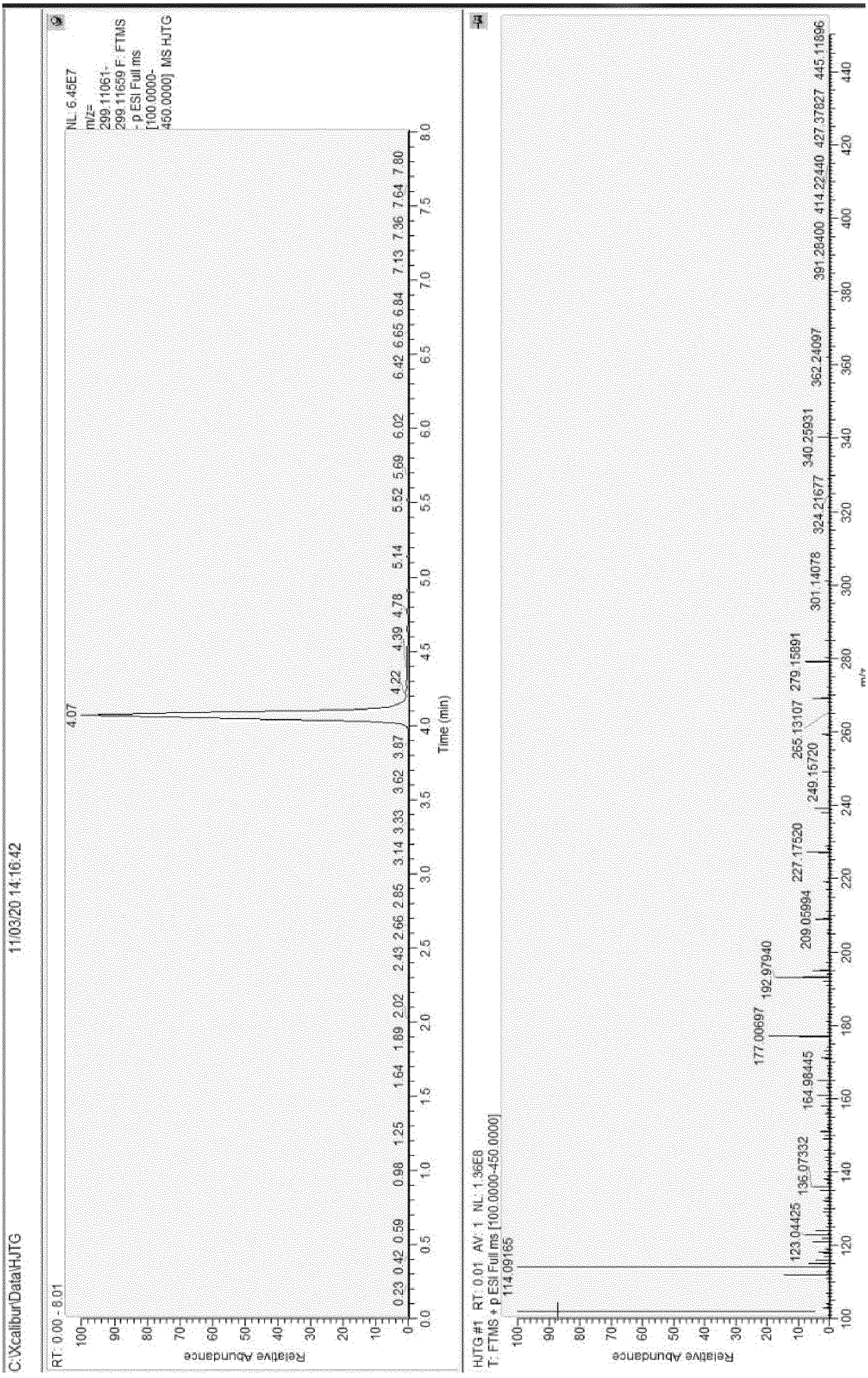
C:\Xcalibur\Data\HJTG
11/03/20 14:16:42
RT: 0.00 - 8.01
NL: 6.45E7
m/z=
299.11061-
299.11659 F: FTMS
- p ESI Full ms
[100.0000-
450.0000] MS HJTG
Relative Abundance
4.07
Time (min)
HJTG #1 RT: 0.01 AV: 1 NL: 1.36E8
T: FTMS + p ESI Full ms [100.0000-450.0000]
114.09165
123.04425
136.07332
164.98445
177.00697
192.97940
209.05994
227.17520
249.15720
265.13107
279.15891
301.14078
324.21677
340.25931
362.24097
391.28400
414.22440
427.37827
445.11896
m/z

FIG. 6

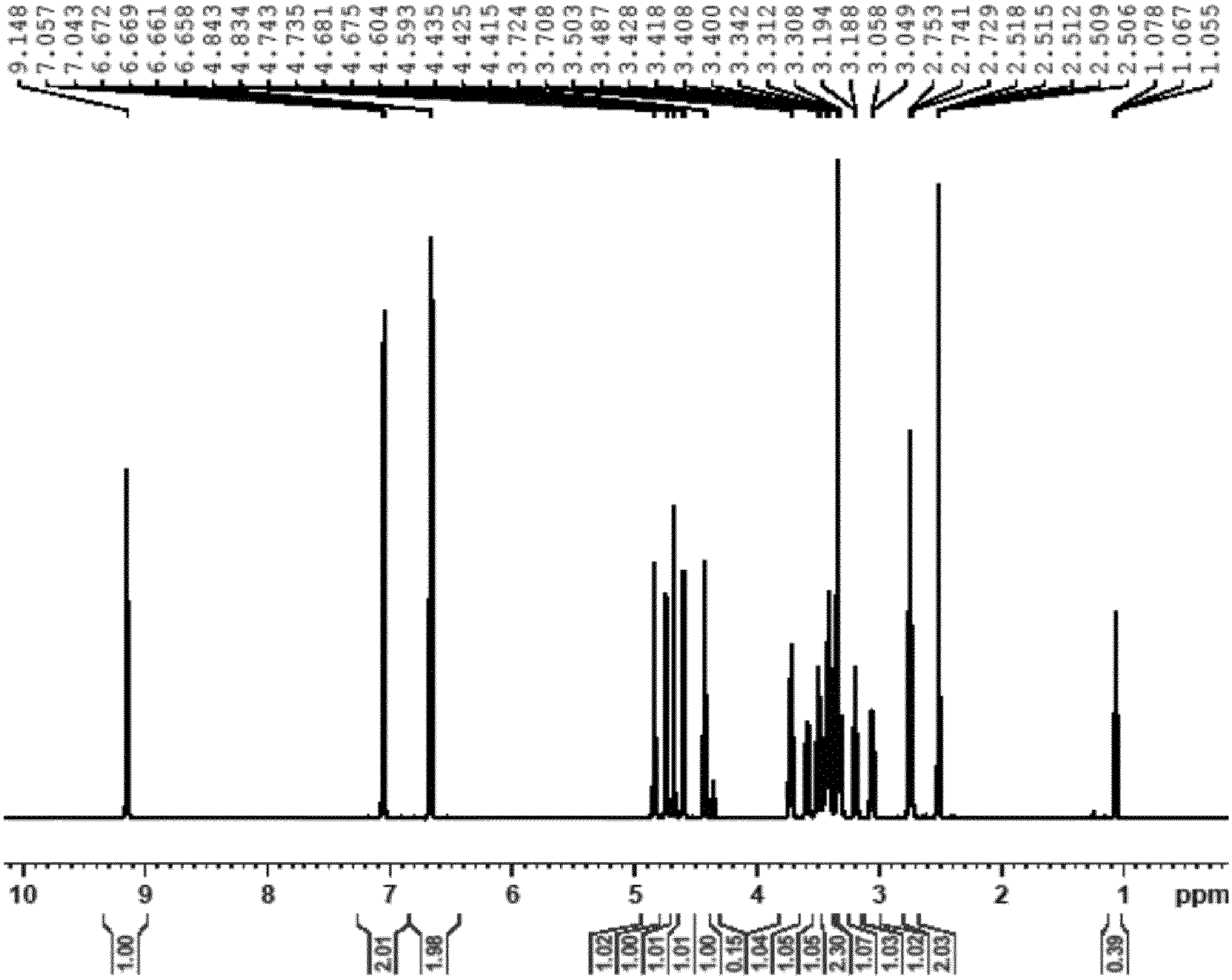
9.148
7.057
7.043
6.672
6.669
6.661
6.658
4.843
4.834
4.743
4.735
4.681
4.675
4.604
4.593
4.435
4.425
4.415
3.724
3.708
3.503
3.487
3.428
3.418
3.408
3.400
3.342
3.312
3.308
3.194
3.188
3.058
3.049
2.753
2.741
2.729
2.518
2.515
2.512
2.509
2.506
1.078
1.067
1.055
10
9
8
7
6
5
4
3
2
1
ppm
1.00
2.01
1.98
1.02
1.00
1.01
1.01
1.00
0.15
1.04
1.06
1.05
2.30
1.07
1.03
1.02
2.03
0.39

# FIG.7

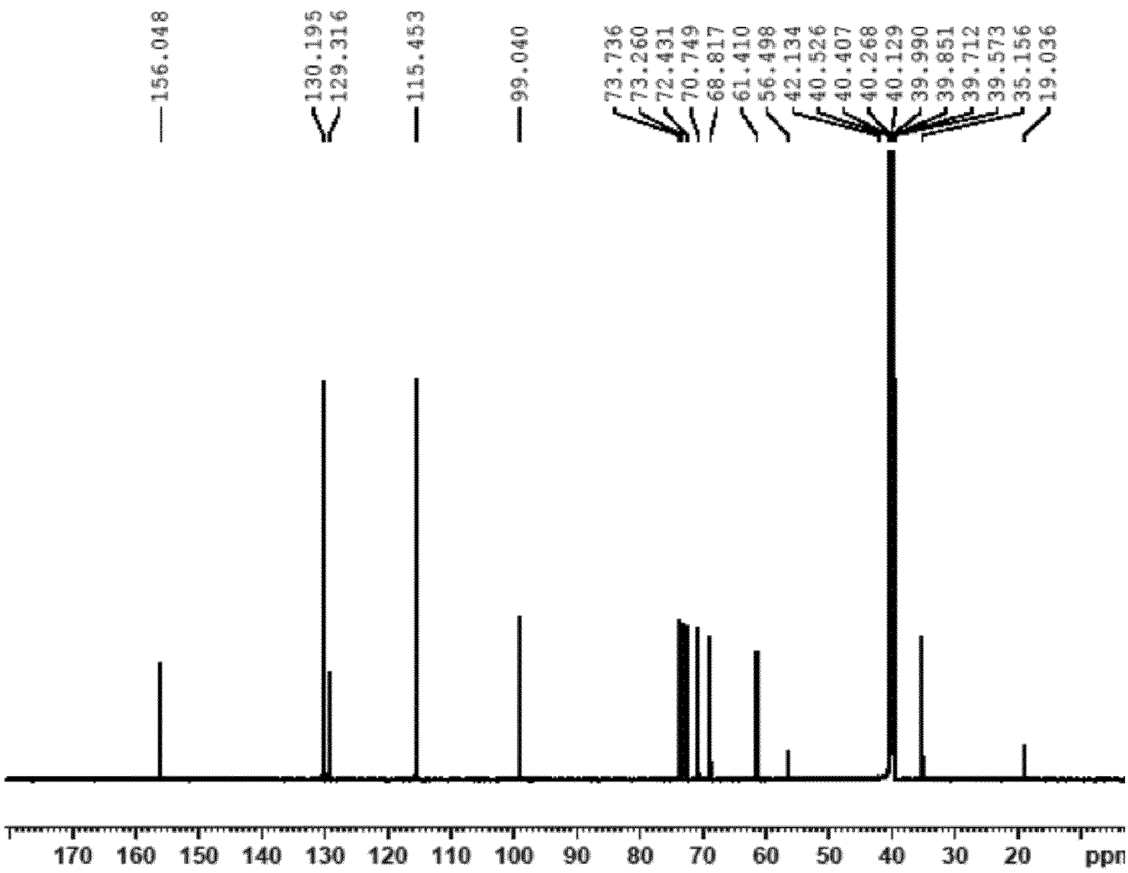
156.048
130.195
129.316
115.453
99.040
73.736
73.260
72.431
70.749
68.817
61.410
56.498
42.134
40.526
40.407
40.268
40.129
39.990
39.851
39.712
39.573
35.156
19.036
170 160 150 140 130 120 110 100 90 80 70 60 50 40 30 20 ppm

FIG. 8

**INTERNATIONAL SEARCH REPORT**

International application No.
**PCT/CN2022/130955**

**A. CLASSIFICATION OF SUBJECT MATTER**

C07H15/18(2006.01)i;C12P19/44(2006.01)i;A61K8/60(2006.01)i;C12R1/06(2006.01)i;C12N9/10(2006.01)i

According to International Patent Classification (IPC) or to both national classification and IPC

**B. FIELDS SEARCHED**

Minimum documentation searched (classification system followed by classification symbols)

IPC:C07H,C12N,C12P,C12R,A61P

Documentation searched other than minimum documentation to the extent that such documents are included in the fields searched

Electronic data base consulted during the international search (name of data base and, where practicable, search terms used)

CNKI, CNABS, CNTXT, DWPI, WOTXT, ENTXT, REGISTRY, CAPLUS: 红景天苷, 对羟基苯乙醇, 酪醇, 葡萄糖苷, 糖基酶, 糖苷酶, 纤维素酶, 淀粉酶, 芳香酶, 转移酶, 酶, 自由基, DPPH, 疲劳, 化妆品, 保健品, 根据权利要求1进行的子结构检索, salidroside, tyrosol, hydroxyphenethyl, glucoside, HPG, enzyme, free radical, cosmetics, health product, glycodidase, glycosylase, cellulase, amylase, aromatase, transferases, fatigue

**C. DOCUMENTS CONSIDERED TO BE RELEVANT**

| Category* | Citation of document, with indication, where appropriate, of the relevant passages | Relevant to claim No. |
|---|---|---|
| X | CN 101347470 A (SHI RENBING) 21 January 2009 (2009-01-21)<br>description, p. 5, line 1, compound | 1 |
| Y | CN 106031708 A (TIANJIN INSTITUTE OF PHARMACEUTICAL RESEARCH CO., LTD.) 19 October 2016 (2016-10-19)<br>description, embodiment 17, and claim 9 | 2-3 |
| Y | CN 104606066 A (SHENZHEN AESTHETIC BIOTECHNOLOGY CO., LTD.; AESTHETIC TECHNOLOGY (BEIJING) CO., LTD.) 13 May 2015 (2015-05-13)<br>description, paragraph 34, and claim 1 | 2-3 |
| Y | CN 101254198 A (INSTITUTE OF MEDICINAL BIOTECHNOLOGY, CHINESE ACADEMY OF MEDICAL SCIENCES) 03 September 2008 (2008-09-03)<br>description, p. 1, technical background section, and p. 2, last paragraph | 2-3 |
| PA | CN 113897406 A (SHANDONG HENGLU BIOTECHNOLOGY CO., LTD.) 07 January 2022 (2022-01-07)<br>entire document | 1-9 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 February 2023** | **27 February 2023** |
| Name and mailing address of the ISA/CN<br>**China National Intellectual Property Administration (ISA/CN)**<br>**China No. 6, Xitucheng Road, Jimenqiao, Haidian District, Beijing 100088**<br>Facsimile No. **(86-10)62019451** | Authorized officer<br><br>Telephone No. |

Form PCT/ISA/210 (second sheet) (July 2022)

**INTERNATIONAL SEARCH REPORT**

International application No. **PCT/CN2022/130955**

**C. DOCUMENTS CONSIDERED TO BE RELEVANT**

| Category* | Citation of document, with indication, where appropriate, of the relevant passages | Relevant to claim No. |
|---|---|---|
| X | TRINCONE, A. et al. "TRINCONE, A. et al." *Bioresource Technology,* Vol. 115, 30 October 2011 (2011-10-30), p. 80, right-hand column, line 1, compound 1a, and p, 80, left-hand column, figure 1 | 1, 4-9 |
| Y | TRINCONE, A. et al. "TRINCONE, A. et al." *Bioresource Technology,* Vol. 115, 30 October 2011 (2011-10-30), p. 80, right-hand column, line 1, compound 1a, and p, 80, left-hand column, figure 1 | 2-3 |
| X | Wang, Qing et al. "A convenient preparation of glycosyl sulfoxides and its application to the synthesis of Salidroside epimer" *Tetrahedron Letters,* Vol. 57, No. 21, 15 April 2016 (2016-04-15), p. 2279, compound 17 | 1 |
| X | 蔡溱等 (CAI, Zhen et al.). "对羟基苯乙基-α-D-葡萄糖甙对小鼠天然杀伤细胞的体外影响 (Non-official translation: Effects of p-Hydroxyphenethyl-α-D-glucoside on Mouse Natural Killer Cells in Vitro)" *中国药理学与毒理学杂志 (Chinese Journal of Pharmacology and Toxicology),* Vol. 12, No. 3, 31 August 1998 (1998-08-31), p. 207, abstract, compound | 1 |
| X | 石力夫等 (SHI, Lifu et al.). "中药女贞子水溶性化学成分的研究 (Studiesonwateer-Soluble Constituents from the Fruitsofligustrum Lucidum Ait)" *药学学报 (Acta Pharmaceutica Sinica),* Vol. 30, No. 12, 31 December 1995 (1995-12-31), p. 937, paragraph 5, compound | 1 |

Form PCT/ISA/210 (second sheet) (July 2022)

**INTERNATIONAL SEARCH REPORT**
**Information on patent family members**

International application No.
**PCT/CN2022/130955**

| Patent document cited in search report | Publication date (day/month/year) | Patent family member(s) | Publication date (day/month/year) |
|---|---|---|---|
| CN 101347470 A | 21 January 2009 | None | |
| CN 106031708 A | 19 October 2016 | None | |
| CN 104606066 A | 13 May 2015 | None | |
| CN 101254198 A | 03 September 2008 | None | |
| CN 113897406 A | 07 January 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202111328730
- CN 202211359878
- CN 104774815 A
- CN 201510160496
- CN 108220264 A
- CN 201611200315
- CN 106543243
- CN 201610979792
- CN 106543244 A
- CN 201610979793
- CN 109321615 A
- CN 201811363071
- CN 102174619 A
- CN 201110005088
- CN 107937457 A
- CN 201711141952


### Non-patent literature cited in the description


- **BASSANINI I ; J KREJZOVÁ ; PANZERI W et al.** A Sustainable One-Pot, Two-Enzyme Synthesis of Naturally Occurring Arylalkyl Glucosides[J]. *Chemsuschem,* 2017, vol. 10, 2040-2045